# EUROPEAN PATENT SPECIFICATION

(11) **EP 1 928 382 B1**
(45) Date of publication and mention of the grant of the patent: **18.11.2009**
(21) Application number: 06815488.9
(22) Date of filing: 26.09.2006
(51) Int. Cl.: A61F 13/20

(54) **TAMPON WITH OVERWRAP AND METHOD OF MANUFACTURE**
TAMPON MIT VERPACKUNG UND HERSTELLUNGSVERFAHREN
TAMPON COMPORTANT UN SUREMBALLAGE ET SON PROCEDE DE FABRICATION

(30) Priority: 26.09.2005 US 720573 P
(43) Date of publication of application: 11.06.2008
(73) Proprietor: The Procter and Gamble Company, Cincinnati, Ohio 45202 (US)
(72) Inventor: BUCK, Travis, Andrew, Sidney, ME 04330 (US); BROAD, Gavin, John, Liberty Township, OH 45011 (US); STRONG, Kevin, Charles, Loveland, OH 45140 (US); BROWN, Gerald, Westley, South Windham, ME 04062 (US)
(74) Representative: Briatore, Andrea
(86) International application number: PCT/US2006/037530
(87) International publication number: WO 2007/038535

(56) References cited:
- GB-A- 2 284 765
- US-A- 3 322 123
- US-A1- 2002 026 177
- US-A1- 2003 097 106

## Description

### FIELD OF THE INVENTION

This invention relates to an improved absorbent catamenial tampon having an ability to readily absorb fluid. This may be accomplished with a tampon made from a compressed absorbent member comprising a novel tampon pledget comprising absorbent material. An overwrap may substantially cover the exterior surface of the compressed absorbent member.

### BACKGROUND OF THE INVENTION

A wide variety of absorbent catamenial tampons have long been known in the art. Most currently commercially available tampons are made from tampon pledgets which have been compressed into substantially cylindrical forms. Tampon pledgets of a variety of types and constructions have been described in the art. Prior to compression, the pledget may be rolled, spirally wound, folded or assembled as a rectangular pad of absorbent material.

Tampons have been constructed from a variety of absorbent materials. These materials have generally included cotton and rayon or homogenous blends of the two materials. These materials are particularly useful in tampon construction because of their low cost, good absorbency, and proven effectiveness in such products.

Tampons may comprise a pledget that may be overwrapped with a liquid permeable non-woven web. The non-woven web, also known as an "overwrap," may provide for various benefits such as a reduction of adhesion of the tampon to vaginal tissue, removal comfort, a clean post-use appearance, and absorption of bypass fluid. An overwrap may also provide for integrity of the tampon pledget as a tampon manufactured without an overwrap may demonstrate an increase in the tendency for rayon and cotton fibers to break off from the tampon pledget.

Generally, an overwrap may be attached to the absorbent material of a tampon pledget. Attaching the overwrap to the absorbent material may enable the overwrap to remain attached to the absorbent material prior to and during attachment of a withdrawal aid. The attachment of the overwrap to the absorbent material may happen after the overwrap is wrapped around the absorbent material and prior to the attachment of a withdrawal aid. Attachment may also provide for an amount of adherence between the tampon pledget and the overwrap after the tampon pledget has been compressed into its final shape. It has now been found, however, that attaching the overwrap over the entirety of the tampon pledget may have a negative impact on the absorbent material.

Attaching the overwrap across the entirety of the absorbent material may compress the absorbent material and may therefore reduce the thickness and therefore increase the density of the absorbent material. The reduction in thickness may increase difficulties in manufacturing a tampon since a denser pledget may be more difficult to fold or compress into a compressed absorbent member.

It has been found that attachment of the overwrap to one or more regions of attachment of the absorbent material rather than to the entirety of the absorbent material produces fewer negative impacts on the absorbent material and its performance. It has been found that attachment of the overwrap to one or more regions of the absorbent material may result in some compressed areas that may allow for an easier attachment of a withdrawal aid. It has also been found that leaving the overwrap unattached to one or more regions of the absorbent material may result in less compressed areas of the absorbent material. Less compressed areas may be thicker, and therefore less dense, and may provide for ease in folding or compressing the pledget into a compressed absorbent member. GB patent application number 2,284,765 describes a rolled tampon comprising an overwrap. US patent 3,322,123 describes a tampon pledget comprising an overwrap which is stitched in the center of the pledget.

It would be desirable to provide a pledget with an overwrap attached to the absorbent material in such a way as to have no negative impact on the absorbent material and its performance. It would be desirable to provide a pledget in which the overwrap is attached to one or more regions of the absorbent material. It would be desirable to provide a pledget with multiple regions of attachment. It would be desirable to provide a pledget with an overwrap attached in such a way as to not have a negative impact on the folding or compression of the pledget.

### SUMMARY OF THE INVENTION

The present invention is related to a tampon pledget comprising absorbent material. The absorbent material may comprise an insertion end, a withdrawal end, a first longitudinal edge, a second longitudinal edge, a first lateral portion, a second lateral portion, a central portion, a first surface and a second surface. An overwrap may substantially cover the first and second surfaces of the absorbent material. The overwrap may be attached to the absorbent material at the central portion in a width greater than about 3 mm. The central portion may comprise a thickness ranging from about 1 mm to about 10 mm.

The overwrap may be attached to the central portion by a method selected from the group consisting of embossing, adhesives, pressure bonding, heat bonding, thermal bonding, fusion bonding, stitching, punching, and combinations thereof.

Considering the pledget in a flat-out, uncompressed state, in one embodiment, the central portion may have a width from about 3 mm to about 30 mm. In one embodiment, each of the first and second lateral portions may have a width from about 5 mm to about 80 mm. In one embodiment, the central portion may comprise less than about 60% of the total width of the absorbent material. Each of the first and second lateral portions may have a thickness from about 5 mm to about 35 mm.

The present invention further relates to a catamenial tampon having an ability to absorb fluid. The tampon may comprise a compressed tampon pledget which comprises an absorbent material and an overwrap. The absorbent material may comprise an insertion end, a withdrawal end, a first longitudinal edge, a second longitudinal edge, a first lateral portion, a second lateral portion, a central portion, a first surface and a second surface. An overwrap may substantially cover the absorbent material. The overwrap may be attached to the absorbent material at the central portion in a width greater than 3 mm. In another embodiment, the tampon may comprise a withdrawal aid. The central portion may comprise a thickness ranging from about 1 mm to about 10 mm.

The present invention also relates to a method of manufacturing a tampon pledget. Absorbent material may be provided comprising an insertion end, a withdrawal end, a first longitudinal edge, a second longitudinal edge, a first lateral portion, a second lateral portion, a central portion, a first surface and a second surface. An overwrap may be provided. The overwrap may be attached to the absorbent material at the central portion in a width greater than 3 mm. Attachment may occur by a method selected from the group consisting of embossing, adhesives, pressure bonding, heat bonding, thermal bonding, fusion bonding, stitching, punching and combinations thereof. In another embodiment, the tampon may comprise a withdrawal aid. In one embodiment, a region of attachment wherein the overwrap is attached to the absorbent material may comprise less than about 75% of the total surface area of the absorbent material.

The present invention also relates to a tampon pledget comprising a compressible absorbent material having a first surface area and a second surface area. The pledget also comprises an overwrap attached to the first surface area and second surface area in a region of attachment. In such an embodiment, the region of attachment may be less than a total surface area of the first and second surface areas.

### BRIEF DESCRIPTION OF THE DRAWINGS

While the specification concludes with claims particularly pointing out and distinctly claiming the subject matter that is regarded as forming the present invention, it is believed that the invention will be better understood from the following description, taken in conjunction with the accompanying drawings, in which:
Figure 1 is a perspective view of a tampon of the present invention.
Figure 2 is a plan view of a tampon pledget of the present invention.
Figure 3 is a plan view of an alternate shape of a tampon pledget of the present invention.
Figure 4 is a plan view of an alternate shape of a tampon pledget of the present invention.
Figure 5 is a plan view of a tampon pledget of the present invention.
Figure 6 is a cross-sectional view of a tampon pledget of the present invention.
Figure 7 is a plan view of an apparatus used to measure the thickness of the absorbent material.
Figure 8 is a plan view of the first and second surfaces of absorbent material and locations on which thickness measurements may occur.

### DETAILED DESCRIPTION OF THE INVENTION

The present invention is directed to an absorbent tampon pledget. The tampon pledget may be compressed to form an absorbent tampon, such as a tampon for menstrual use.

The term "compressed," as used herein, refers to pressing or squeezing together or otherwise manipulating the size, shape, and/or volume to obtain a generally elongated absorbent member having a vaginally insertable shape.

As used herein, "fluid wicking" refers to the ability of a material to carry fluid or moisture by capillary action. The fluid wicking capacity of a medium can be measured by grams of fluid drawn per gram of tampon weight over a fixed period of time.

The term "attached," as used herein, encompasses: configurations in which a first element may be directly secured to a second element by affixing the first element directly to the second element; configurations in which the first element may be indirectly secured to the second element by affixing the first element to an intermediate member which in turn may be affixed to the second element; and configurations in which the first element may be integral with a second element, i.e., the first element may be essentially part of the second element.

As used herein, "overwrap" refers to the liquid pervious material covering the exterior surface of the absorbent member. The overwrap may permeate the inner region of a compressed absorbent member. The overwrap may extend below the withdrawal end to form a skirt portion. The overwrap may be fluid wicking. The overwrap, as defined herein, may possess a horizontal wicking capacity of at least about 2, alternatively from about 3 to about 6 grams of fluid per gram of tampon at a 500 second interval. Suitable overwraps are disclosed in greater detail in US Patent No. 6,840,927, US Patent Publication Nos. 2003/0097104 and 2003/0097106.

The terms "pledget" and "tampon pledget" refer herein to a construction of absorbent material prior to the compression of such construction into a tampon as described below. Tampon pledgets are sometimes referred to as tampon "blanks," or a "softwind," and the term "pledget" is intended to include such terms as well.

The term "tampon," as used herein, refers to any type of absorbent structure that is inserted into the vaginal cavity or other body cavities for the absorption of fluid therefrom. Typically, tampons are constructed from a generally elongated absorbent member that has been compressed and/or formed into a vaginally insertable shape.

The term "vaginal cavity" refers herein to the internal genitalia of the human female in the pudendal region of the body. The term "vaginal cavity" as used herein is intended to refer to the space located between the introitus of the vagina (sometimes referred to as the sphincter of the vagina) and the cervix and is not intended to include the interlabial space, including the floor of the vestibule. The external visible genitalia generally are not included within the term "vaginal cavity" as used herein.

The term "vaginally insertable shape" as used herein refers to the geometrical form of the absorbent tampon after compression. The tampon may be compressed into a generally cylindrical configuration in the radial direction along the longitudinal and/or lateral axes, axially, or in both the radial and axial directions. An example of a typical compressed tampon may be one which may be about 10-16 mm wide and about 40- 50 mm long depending on the level of absorbency. While the tampon may be compressed into a substantially cylindrical configuration, other shapes are possible. These may include shapes having a cross section that may be described as rectangular, trapezoidal, seni-circular, hourglass, or other suitable shapes.

The term "volume" refers herein to the volume of the fibers and the void space within the pledget. Volume is measured by the multiplication of the length by the width by the thickness of the pledget.

As used herein, "cm" is centimeter, "mm" is millimeter, "g" is gram, "gsm" is grams per meter squared, "dpf" is denier per fiber, "g/g" is gram of fluid per gram of sample, "wt" is weight, "psi" is pound per square inch.

While particular embodiments of the present invention have been illustrated and described, it would be obvious to those skilled in the art that various other changes and modifications can be made without departing from the spirit and scope of the invention.

### Tampon of the Present Invention

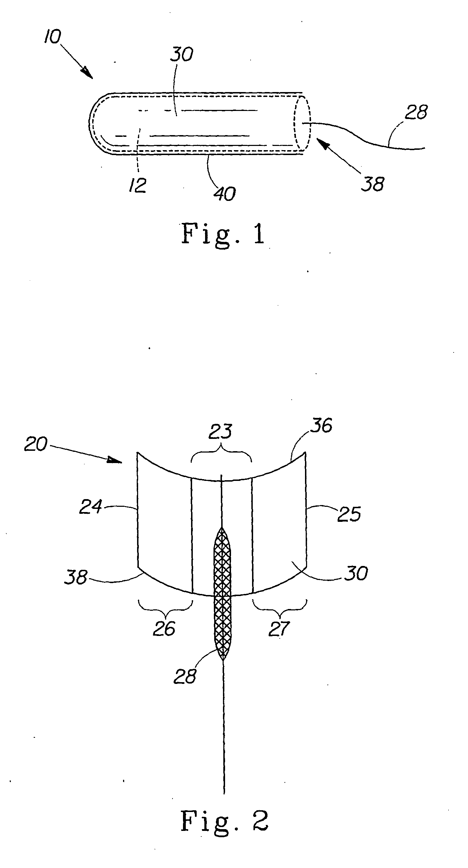

Figure 1 exemplifies a finished tampon 10 made from a pledget of the present invention. The present invention, however, is not limited to a structure having the particular configuration shown in the drawings. The tampon 10 may comprise a compressed absorbent member 12 comprising absorbent material 30, and an overwrap 40 that substantially covers the absorbent material 30 of the compressed absorbent member 12. In one embodiment, the tampon 10 may include a withdrawal aid 28 extending beyond the withdrawal end 38.

### Absorbent Material

Figure 2 exemplifies a tampon pledget 20 in a flat-out, uncompressed state. The absorbent material 30 of the present invention forms a tampon pledget 20. The tampon pledget 20 comprises an insertion end 36, a withdrawal end 38, a first longitudinal edge 24, a second longitudinal edge 25, a first lateral portion 26, and a second lateral portion 27. A central portion 23 is situated between the first lateral portion 26 and the second lateral portion 27. A withdrawal aid 28 may be attached to the absorbent material 30. Compression of a tampon pledget 20 may form a compressed absorbent member 12 (as shown in Figure 1). A compressed absorbent member 12 in combination with a withdrawal aid 28 may form a tampon 10 (as shown in Figure 1).

The tampon pledget 20 may be any suitable shape, size, material, or construction for compression or formation into a tampon 10 having a vaginally insertable shape. The absorbent material 30 may be generally square or rectangular or take on other shapes such as trapezoidal, triangular, hemispherical, chevron or hourglass shapes.

As Figure 2 exemplifies, the tampon pledget 20 may be a batt of absorbent material 30 which may be a generally "chevron shaped" pad of absorbent material 30. While a chevron shaped pledget 20 may be suitable, the edges of the chevron may be somewhat "rounded off" in order to facilitate high speed manufacturing operations. While the pledget 20 shown in Figure 2 is generally chevron shaped, other shapes such as trapezoidal, triangular, semi-circular, and rectangular shaped are also acceptable.

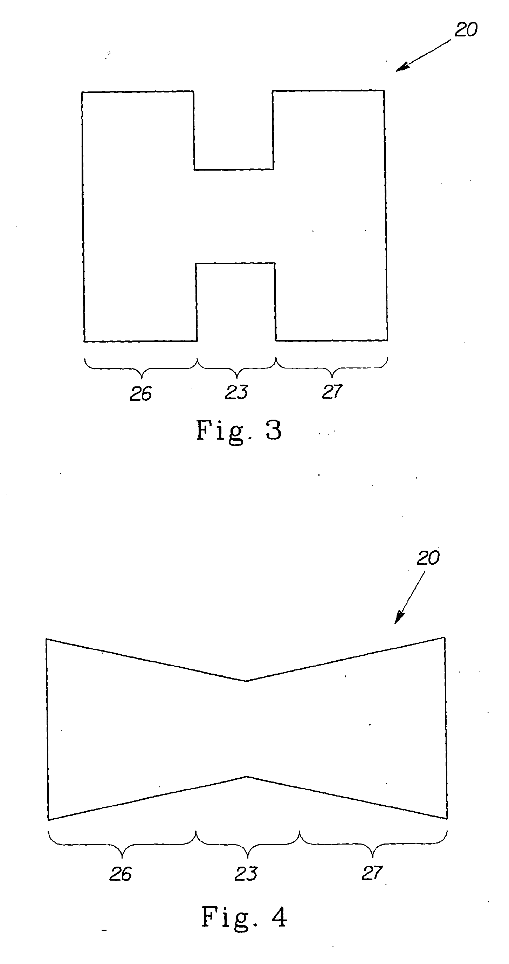

Other shapes may also be possible. For example, the pledget 20 may be generally "H" shaped, such as shown in Figure 3. A "bow tie" shaped tampon pledget 20, such as is shown in Figure 4, is also suitable. As an alternative to the shapes of pledgets 20 described above, a tampon pledget 20 of the present invention may have a uniform shape such as a rectangular shape, but may vary in absorbent material 30 thickness along the axial extent of the pledget 20.

The tampon pledget 20 may be constructed from a wide variety of liquid-absorbing materials commonly used in absorbent articles such as rayon (including tri-lobal and conventional rayon fibers), cotton, or comminuted wood pulp which is generally referred to as airfelt. Examples of other suitable absorbent materials include, but are not limited to, creped cellulose wadding; meltblown polymers including coform; chemically stiffened, modified or cross-linked cellulosic fibers; synthetic fibers such as crimped polyester fibers; peat moss; foam; tissue including tissue wraps and tissue laminates; or any equivalent material or combinations of materials, or mixtures of these.

Typical absorbent materials may comprise cotton, rayon folded tissues, woven materials, non-woven webs, synthetic and/or natural fibers or sheeting. The tampon 10 and any component thereof may comprise a single material or a combination of materials. Additionally, superabsorbent materials, such as superabsorbent polymers or absorbent gelling and open-celled foam materials may be incorporated into the tampon 10.

In another non-limiting embodiment, the absorbent material 30 and resulting compressed absorbent member 12 (shown in Figure 1) may comprise rayon, cotton, or combinations of both materials. These materials have a proven record of suitability for use in the human body. The rayon used in the absorbent material 30 may be any suitable type typically used in disposable absorbent articles intended for in vivo use. Such acceptable types of rayon include GALAXY Rayon (a tri-lobed rayon structure) available as 6140 Rayon from Acordis Fibers Ltd., of Hollywall, England. SARILLE L rayon (a round fiber rayon), also available from Acordis Fibers Ltd. is also suitable. Any suitable cotton material may be used in the absorbent material 30. Suitable cotton materials include, long fiber cotton, short fiber cotton, cotton linters, T-fiber cotton, card strips, and comber cotton. The cotton may be scoured and bleached cotton absorbent with a glycerin finish, or other suitable finish.

The absorbent material 30 maybe a laminar structure comprised of integral or discrete layers. If the compressed absorbent member 12 of the present invention is layered, the layers may comprise different materials. For example, in one embodiment, the outer layers may comprise primarily rayon, while the intermediate layer or layers may comprise primarily cotton. Optionally, the entire compressed absorbent member 12 may comprise a uniform or non- uniform blend of materials throughout. The absorbent material may comprise 100% rayon fibers or 100% cotton fibers. The absorbent material may comprise a combination of rayon and cotton fibers in any suitable combination. The absorbent material may comprise greater than about 25%, 30% or 40% rayon fibers and the balance of the absorbent material comprising cotton fibers. The absorbent material of the present invention may comprise greater than about 50% rayon fibers with cotton fibers comprising the balance of the absorbent material. In another embodiment, the absorbent material may comprise greater than about 60, 70, 75, 80 or 90 % rayon fibers and the balance of the absorbent material comprising cotton fibers. In one layered embodiment, each of the layers may comprise essentially 100% of the same material, such as outer layers of 100% rayon and an intermediate layer of 100% cotton. A Super Plus absorbency tampon of the present invention may be made from a pledget comprising about 100% rayon fibers. A Super absorbency or Regular absorbency tampon of the present invention maybe made from a pledget comprising about 25% cotton and about 75% rayon fibers. A Junior absorbency tampon may be made from a pledget comprising about 50% cotton and about 50% rayon fibers.

A typical size for absorbent material 30 prior to compression may be from about 30 or 40 mm to about 60, 70, 80, 90 or 100 mm in length and from about 40 or 50 mm to about 70, 75, 80, 85, or 90 mm in width. The typical range for the overall basis weight may be from about 150, 200, or 250 gsm to about 600, 800, 1000 or 1100 gsm.

### Overwrap

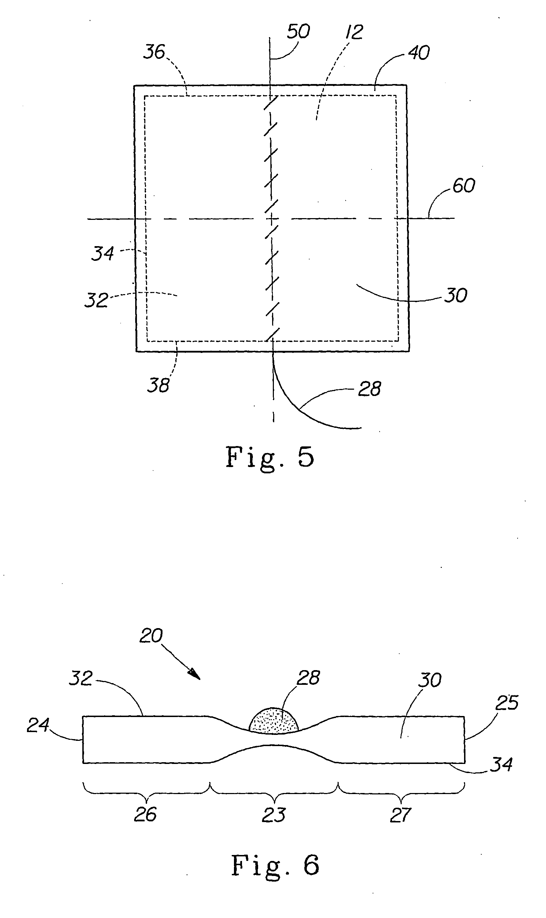

Figure 5 exemplifies the overwrap 40 substantially covering the absorbent material 30 prior to compression. The absorbent material 30 may have a first surface 32 opposed to a second surface 34. The absorbent material 30 may have a longitudinal axis 50 and a transverse axis 60.

In the embodiment shown in Figure 5, the overwrap material 40 is generally rectangular, but other shapes such as trapezoidal, triangular, hemispherical, chevron, hourglass shaped, "T" and "L" shaped are also acceptable. Optimally, the overwrap 40 may correspond to the shape of the absorbent material 30. The overwrap 40 may be positioned around the absorbent material 30 so that the overwrap 40 maybe proximate with the insertion end 36 of the absorbent material 30. In this regard, the overwrap 40 could exactly match up to the insertion end 36 or could, for example, extend from about 2 mm to about 8 mm over the insertion end. As well, the overwrap 40 may be proximate with the withdrawal end 38. In another embodiment, the overwrap 40 may extend from the withdrawal end 38 from about 2 mm to about 8 mm to form a skirt portion (not shown).

Because the overwrap 40 can be wrapped in the various configurations, the width of the overwrap 40 may vary. The width of the overwrap 40 may be wider or less wide than the measure of the longitudinal axis 50 or transverse axis 60 of the absorbent material 30 it is being wrapped around.

The overwrap 40 may substantially cover both the first surface 32 and the second surface 34 of the absorbent material 30 of the pledget. "Substantially cover" in this case means that the overwrap 40 covers at least about 50%, optionally at least about 75% or even 90% of the combined surface area of the first surface 32 and the second surface 34. Thus, for example, the overwrap 40 "substantially covers" the first surface 32 and the second surface 34 of the absorbent material 30 when it covers 100% of the first surface 32 and 50% of the second surface 34. As well, two separate pieces of overwrap 40 can form a laminate and may cover the absorbent material 30.

The overwrap may be attached to the absorbent material in at least one region of attachment. The at least one region of attachment may be less than the total surface area of the absorbent material. The at least one region of attachment may comprise less than about 75% of the total surface area of the absorbent material. In another embodiment, the at least one region of attachment may comprise less than about 50% of the total surface area of the absorbent material. In yet another embodiment, the at least one region of attachment may comprise less than about 40% of the total surface area of the absorbent material. The absorbent material may comprise a central portion 23 and first and second lateral portions, 26 and 27 (shown in Figure 2). The at least one region of attachment may be located in the central portion 23, first lateral portion 26, second lateral portion 27, and combinations thereof. The overwrap may be attached in the central portion in a region of attachment in a width that is greater than about 3 mm. In another embodiment, the overwrap may be attached in the central portion in a region of attachment in a width that is greater than about 5, 7, 9, 10, 12 or 15 mm. In another embodiment, there may be more than one region of attachment located in the central portion 23, first lateral portion 26, second lateral portion 27 and combinations thereof. In another embodiment, the overwrap may be attached to the first and second surface areas of the absorbent material of the pledget in at least one region of attachment. In such an embodiment, the at least one region of attachment may be less than the total surface area of the first and second surface areas. Overwrap extending beyond the at least one region of attachment may be unattached to the absorbent material. Attachment may be accomplished by adhesives, heat bonding, pressure bonding, thermal bonding, fusion bonding, stitching, embossing, punching, or any other suitable means known in the art and combinations thereof.

In one embodiment, the overwrap may be embossed onto the at least one region of attachment. Embossing rolls may be utilized to attach the overwrap to the absorbent material. The embossing rolls may be smooth or may comprise any desired pattern of at least one protuberance or recessed area. Such protuberance may extend from the embossing rolls in either a uniform or non-uniform thickness. In one embodiment, there may be more than one pattern of protuberance or recessed area. In another embodiment in which smooth embossing rolls may be utilized, the attachment of the overwrap to the absorbent material may result in a uniform thickness in a region of attachment. In yet another embodiment in which patterned embossing rolls, or otherwise comprising at least one protuberance or recessed area, may be utilized, the attachment of the overwrap to the absorbent material may result in a region of attachment comprising varying thicknesses of absorbent material. In yet another embodiment in which embossing rolls are utilized, the attachment of the overwrap to the first and second surfaces of the absorbent material may result in a smooth or patterned embossment, or combinations thereof, of the first and second surfaces of the absorbent material. For example, a first surface of the embossed pledget may comprise a patterned embossment in the central portion of the first surface produced by an embossing roll comprising at least one protuberance and the second surface of the embossed pledget may comprise a different patterned embossment which may be in a region of attachment in both the central portion and at least one of the lateral portions of the second surface produced by an embossing roll comprising a different pattern of at least one protuberance. Alternatively, the second surface of the embossed pledget just described may be smooth as produced by a smooth embossing roll.

In one embodiment in which regions of attachment may be in both the central portion and at least one lateral portion, the thickness of the absorbent material in the region of attachment in the central portion may be less than the thickness of the absorbent material in the region of attachment in the lateral portion.

Attachment of the overwrap to the absorbent material may provide benefits for the overall structure of the absorbent material. Attachment of the overwrap to the absorbent material may produce an area of decreased thickness within the region of attachment. Portions of the absorbent material outside the regions of attachment may provide areas of higher thickness of absorbent material. A benefit of decreased thickness that may be realized is an ease of attaching a withdrawal aid to the absorbent material. Attachment of a withdrawal aid by a method such as stitching may benefit from a decrease in the thickness of the absorbent material. A decrease in thickness of the absorbent material may provide for a lower loft of the absorbent material thus allowing for an ease with which a stitching mechanism, such as a needle, may pass through the absorbent material.

The overwrap may comprise a fibrous liquid permeable non-woven material comprising a blend of synthetic and natural fibers. The synthetic fibers may include, but are not limited to, fibers such as polyester, polyolefin, nylon, polypropylene, polyethylene, polyacrylic, cellulose acetate or bicomponent fibers. Natural fibers may include, but are not limited to, those commonly known to be non-synthetic and of natural origin such as cotton and/or rayon. In general, the natural fibers may provide ready absorption and fluid wicking strength. The synthetic fibers may balance the capillary strength of the blended material, enabling the tampon to more readily slip against moist tissue, resulting in easier removal and hence removal comfort. The ratio of synthetic fibers to natural fibers may fall in the range of from about 90:10 to about 30:70. Alternatively, the ratio of synthetic fibers to natural fibers may fall in the range of from about 70:30 to about 40:60. The synthetic fibers may have hydrophobic and/or hydrophilic surfaces. The synthetic fibers may be inherently hydrophilic, or may preferably be treated to provide such properties. The overwrap may comprise some level of hydrophobic fibers as well, as long as it does not significantly diminish the fluid wicking capacity of the overwrap of the tampon.

The blend of fibers forming the overwrap can be made by any number of techniques. The blends may be carded on webs. Commonly, carded webs that are hydroentangled, thermally bonded, and resin bonded all have application. In the latter case, the resin bonding agent can be used in place of the synthetic fibers as the method for tempering the aggressiveness of the natural fiber matrix. In this case, all natural fiber may be used with a significant amount of synthetic binder (10-30% by weight is common). Spunbond and meltblown processes, combining synthetic fibers extruded/spun onto/into a mat or carded web of natural fibers provide other acceptable techniques. The basis weight of the overwrap may fall into a range from about 10, 12 or 15 grams per square meter to about 30, 40, 50 or 60 grams per square meter.

### Optional Components

Optionally, the tampon 10 of the present invention may include an additional overwrap. This additional overwrap may substantially cover the overwrap 40 that substantially covers the exterior surface 16 of the compressed absorbent member 12 of the tampon 10. This additional overwrap may be added prior to or subsequent to compression of the absorbent material to form a compressed absorbent member.

As exemplified in Figure 5, in one embodiment, the tampon 10 of the present invention may comprise a withdrawal aid 28. The withdrawal aid 28 may be attached to the absorbent material 30 and may extend beyond at least the withdrawal end 38. Any of the withdrawal aids currently known in the art may be used as a suitable withdrawal aid. In addition, the withdrawal aid 28 can take on other forms such as a ribbon, loop, tab, or the like. The withdrawal aid 28 may be integral with the absorbent material 30.

The withdrawal aid 28 may be non-absorbent along at least the location of attachment to the absorbent material 30. As used herein, the term "non-absorbent" refers to a structure that does not retain a significant portion of deposited fluid in its structure. The entire withdrawal aid 28 may be made non-absorbent, if desired. The materials comprising the withdrawal aid 28 may be inherently non-wettable or hydrophobic, or they may be treated to provide such properties. For example, a coating of wax may be applied to the withdrawal aid 28 to decrease or eliminate its absorbency. The withdrawal aid 28 need not necessarily be non-wicking, even if a non-absorbent withdrawal aid 28 is desired.

The withdrawal aid 28 may be attached to the absorbent material in any suitable manner known in the art including sewing, adhesive attachment, or a combination of known bonding methods. The withdrawal aid 28 may be attached to any suitable location on the absorbent material 30. Additional suitable withdrawal aids and attachment mechanism may be found in US Patent Publication No. 2004/0261237.

The tampon 10 of the present invention may be inserted digitally or through the use of an applicator. Any of the currently available tampon applicators may also be used for insertion of the tampon 10 of the present invention. Such applicators of typically a "tube and plunger" type arrangement and may be plastic, paper, or other suitable material. Additionally, a "compact" type applicator is also suitable.

### Process of Making

While several methods of making the tampon of the present invention should be apparent to one of skill in the art in light of the disclosure herein, the following may be one embodiment of one method of making a tampon of the present invention.

The process for making a tampon may comprise the steps of providing an absorbent material having a first surface opposed to the second surface and an insertion end opposed to a withdrawal end**.** An overwrap is also provided. A wrapped absorbent may be created by substantially covering the first surface and second surface of the absorbent material with the overwrap. In some embodiments, the overwrap may extend beyond the withdrawal end of the absorbent material to form a skirt portion. The process may include providing a withdrawal aid that may be attached to the absorbent material.

The materials for the absorbent material can be formed into a fabric, web, or batt that is suitable for use in the absorbent material by any suitable process such as airlaying, carding, wetlaying, hydroentangling, needling or other known techniques.

The overwrap may be attached to the absorbent material by any variety of methods. One portion of the overwrap may be attached to the absorbent material using any suitable adhesive, heat bonding or pressure bonding. Such adhesive may extend continuously along the length of attachment or it may be applied in an intermediate or a "dotted" fashion at discrete intervals. Other methods of attachment may include heat bonding, thermal bonding, fusion bonding, embossing, pressure bonding or any other suitable means known in the art for joining such materials. Alternatively, the overwrap may be attached to the absorbent material along by stitching or punching. Such stitching or punching may use natural or synthetic thread.

The attachment of the overwrap to the absorbent material may form at least one region of attachment. A region of attachment may be located in the absorbent material in a central portion, first lateral portion, second lateral portion and combinations thereof. In one embodiment, a region of attachment may be in the central portion of the absorbent material. In such an embodiment, the region of attachment may be the entire central portion or may be less than the central portion. The region of attachment may be in a width of greater than about 3, 5, 7, 9, 10, 12 or 15 mm. The region of attachment may be in a width of from about 3 mm to about 30 mm or any other number encompassed within that range. In another embodiment, the overwrap may be attached to the absorbent material in the central portion and at least one lateral portion. In such an embodiment, the region of attachment in the central portion may be the entire central portion or may be less than the central portion. A region of attachment in at least one lateral portion may be the entire lateral portion or may be less than the lateral portion. In such an embodiment, attachment of the overwrap to the absorbent material in a region of attachment may occur by more than one method of attachment, such as, in the central portion attachment may be by embossing and attachment of the overwrap in at least one lateral portion may utilize any suitable adhesive. In another embodiment, attachment in the central portion may utilize one embossing pattern and attachment in at least one lateral portion may utilize a second embossing pattern.

In one embodiment, the overwrap may be embossed onto the absorbent material. The embossing rolls may generally be heated and pressurized along the center of the embossing rolls. As the absorbent material and overwrap pass between the embossing rolls, the area of pressure may be applied to the region of attachment. The overwrap may, therefore, be embossed onto at least one region of attachment of the absorbent material. Absorbent material outside of at least one region of attachment may remain unembossed by the overwrap; however, the unembossed absorbent material remains covered by the overwrap. The embossing rolls may be smooth or may comprise any desired pattern or at least one protuberance. Such pattern or protuberance may extend from the embossing rolls in either a uniform or non-uniform thickness. In one embodiment, there may be more than one pattern or protuberance. In another embodiment in which smooth embossing rolls are utilized, the attachment of the overwrap to the absorbent material may result in a uniform thickness in a region of attachment. In yet another embodiment in which patterned embossing rolls, or otherwise comprising at least one protuberance, are utilized, the attachment of the overwrap to the absorbent material may result in a region of attachment comprising varying thicknesses of absorbent material.

Figure 6 exemplifies a cross-sectional view of a tampon pledget 20. The central portion 23 may have a width from about 3 mm to about 15, 20 or 30 mm. In one embodiment, the central portion 23 may comprise less than about 60% of the total width of the absorbent material 30. In another embodiment, the central portion 23 may comprise less than about 50% of the total width of the absorbent material 30. In yet another embodiment, the central portion 23 may comprise less than about 40% of the total width of the absorbent material 30. The central portion 23 may have a thickness from about 1 or 2 mm to about 7 or 10 mm. The thickness of the central portion 23 is measured with a gauge that measures the thickness of the absorbent material 30 between the first and second surfaces, 32 and 34, constituting the central portion 23 after the overwrap 40 is attached to the absorbent material 30. The method for measuring the thickness of the central portion is described below.

Each of the first and second lateral portions, 26 and 27, may have a width from about 5, 10, 15 or 20 mm to about 60, 70 or 80 mm. Each of the first and second lateral portions, 26 and 27, may have a thickness from about 5 or 7 mm to about 10, 15, 20, 25, 30 or 35 mm. The thickness is measured by a gauge that measures the thickness of the absorbent material 30 between the first and second surfaces, 32 and 34, constituting the lateral portions, 26 and 27, after the overwrap 40 is attached to the absorbent material 30. The method for measuring the thickness of the lateral portions is described below.

The thickness of the lateral portions may be greater than the thickness of the central portion. The difference in thickness may be expressed as a ratio of the lateral portion to the central portion. The ratio of the thickness of the lateral portions to the central portion may be greater than about 1.20:1, 1.40:1, 1.50:1, 1.75:1, or 2.00:1. In another embodiment, the ratio of the thickness of the lateral portions to the central portion may be greater than about 2.10:1, 2.25:1, 2.50:1, 2.75:1, 3.00:1, 3.25:1, or 3.50:1. The ratio of the thickness of the lateral portion to the central portion may be from about 1.20:1, 1.40:1, 1.50:1, or 1.75:1 to about 2.00:1, 2.50:1, 3.00:1, or 3.50:1 or any ratio within the range.

The tampon pledget may be folded and/or compressed to form a compressed absorbent member having a vaginally insertable shape. Pressures and temperatures suitable for compression are well known in the art. Topically, the absorbent material and the overwrap may be compressed in the radial direction and optionally axially by any means well known in the art. While a variety of techniques are known and acceptable for these purposes, a modified tampon compressor machine available from Hauni Machines, Richmond, Va., maybe suitable. Upon compression the overwrap may substantially cover the exterior surface of the compressed absorbent member and may permeate into the interstices of the inner region of the compressed absorbent member.

### Exemplary Pledgets

In one embodiment, an example of a Super Plus absorbency pledget made according to the present invention may be about 70mm in width and about 48mm in length. Such a pledget may include 100% Rayon fibers. An overwrap may be attached in a region of attachment within the central portion. The central portion of the pledget may be about 3mm in thickness and the lateral portions of the pledget may be about 7 mm in thickness. The volume of the central portion maybe about 10.08cc and the volume of the lateral portions maybe about 23.52 cc. The weight of the pledget maybe about 3.26g.

In another embodiment, an example of a Lite absorbency pledget made according to the present invention may be about 40 mm in width and about 37 mm in length. Such a pledget may include 75% Rayon fibers and 25% cotton fibers. An overwrap may be attached in a region of attachment within the central portion. The central portion of the pledget may be about 3 mm in thickness and the lateral portions of the pledget maybe about 7 mm in thickness. The volume of the central portion may be about 4.44cc and the volume of the lateral portions may be about 10.36cc. The weight of the pledget may be about 1.2g.

In yet another embodiment, an example of a Regular absorbency pledget made according to the present invention may be about 70mm in width and about 46mm in length. Such a pledget may include 50% Rayon fibers and 50% cotton fibers. An overwrap may be attached in a region of attachment within the central portion. The central portion of the pledget may be about 2mm in thickness and the lateral portions of the pledget may be about 5mm in thickness. The volume of the central portion may be about 6.44 cc and the volume of the lateral portions may be about 16.1 cc. The weight of the pledget may be about 1.85g.

It should be understood that these are examples of a pledget made according to the present invention and multiple variations may be made in the width, length, thickness and density as discussed above and claimed below.

### Test Methods

### Thickness Measurement

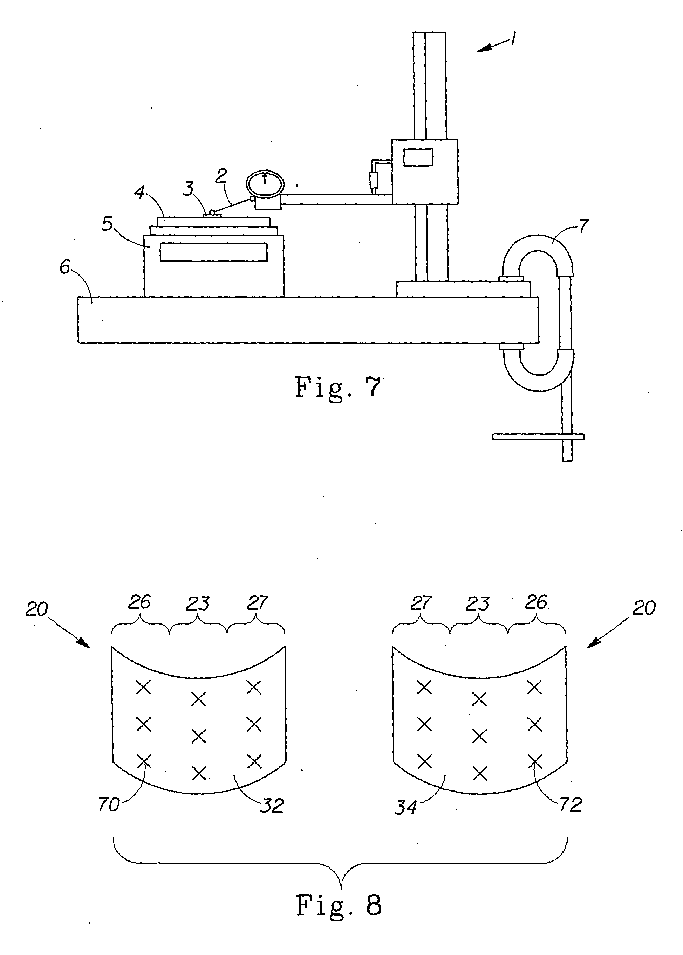

The thicknesses of the central portion and lateral portions of the absorbent material comprising an overwrap are measured using the method described below. Uncompressed absorbent material is preconditioned at 73.4°F ± 2° and 50% ± 5% RH for at least 4 hours prior to analysis. Measurements are then performed at 73.4°F ± 2° and 50% ± 5% RH conditions. On the flat uncompressed absorbent material, several positions along the lateral portions and central portion of the absorbent material are measured for thickness on both surfaces of the absorbent material, as shown in Figure 8. The lateral portions and central portion absorbent material thickness are then calculated as described in the method below.

The equipment for this procedure is shown in Figure 7.
Mitutoyo Digimatic Height Gauge HDS-8"M
Puppitast 800SL Stylus Gauge
Sartorius 4200 S Balance or equivalent
5 mm diameter non-deformable plastic disk weighing about 0.05 g or less Clamp
Balance Table

A height gauge 1 is placed on a balance table 6 and clamped down 7. A balance 5 is placed on the balance table 6. The sample 4 is placed on the balance 5 with the 5 mm disk 3 placed on the sample 4. The stylus gauge 2 is placed on the 5 mm disk 3.

### Procedure:

1. With the Mitutoyo gauge raised and nothing on the balance pan, tare the balance.
2. Place the 5 mm diameter non-deformable plastic disk on the balance pan.
3. Manually lower the Mitutoyo gauge until the Puppitast Stylus just contacts the top of the disk.
4. The load on the balance should be about 1 g.
5. Use the fine adjust so that the load on the balance is 1.5 g +/- 0.2g.
6. Make sure the Puppitast gauge is reading between 0.01 and 0.03 mm.
7. Zero the Mitutoyo gauge.
8. Raise the Mitutoyo gauge and remove the disk off the balance.
9. Place a flat uncompressed absorbent material on the balance and tare the balance.
10. Place the disk on the sample above the area where the thickness is to be measured (as exemplified in Figure 8 at locations such as 70 and 72 on the first surface 32 and second surface 34, respectively).
11. Carefully lower the Mitutoyo gauge until the stylus touches the disk.
12. Finely adjust the height to give a load on the balance of 1.5 g +/- 0.2g.
13. Read the thickness from the Mitutoyo gauge.
14. Repeat steps 10 -13 for a total of 3 thickness measurements along each lateral portion and central portion on each surface of the flat uncompressed absorbent material. Each area of thickness measurement should be equidistant in the latitudinal direction and equidistant in the longitudinal direction from the four nearest entities whether the nearest entity is an edge of the flat uncompressed absorbent material or another area of thickness measurement.

### Calculation:

The lateral portion thickness of the absorbent material is calculated by averaging all of the thickness measurements of the lateral portion of the absorbent material on both surface of the absorbent material.

The thickness of the central portion of the absorbent material is calculated using the following approach:
1. Average the thickness measurements collected from the central portion of the absorbent material from the first surface.
2. Average the thickness measurements collected from the central portion of the absorbent material from the second surface.
3. Add together the average central portion thicknesses from the first and second surfaces and then subtract this number from the average lateral portion thickness of the absorbent material calculated above to arrive at the thickness of the central portion.

The citation of any document is'not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

## Claims

1. A tampon pledget (20) comprising
a. an absorbent material (30) which comprises a first surface, a second surface opposed to said first surface, an insertion end (36) opposed to a withdrawal end (38), a first longitudinal edge (24) opposed to a second longitudinal edge (25), a first lateral portion (26) opposed to a second lateral portion (27), and a central portion (23) between said first lateral portion (26) and said second lateral portion (27), and wherein a thickness of said central portion (23) ranges from 1 mm to 10 mm, said absorbent material (30) having a length of from 30 to 100 mm, a width of from 40 to 90 mm and a basis weight of from 250 to 1100 gsm; and
b. an overwrap (40) that covers said first surface of said absorbent material (30),
said tampon pledget (20) being **characterized in that** said overwrap (40) is attached to said central portion (23) in a width greater than 3 mm.

2. The tampon pledget (20) of Claim 1 wherein said overwrap (40) is attached to said central portion (23) by a method selected from the group consisting of embossing, adhesive, pressure bonding, heat bonding, thermal bonding, fusion bonding, stitching, and combinations thereof.

3. The tampon pledget (20) of any of the preceding claims wherein a width of said central portion (23) ranges from 3 mm to 30 mm.

4. The tampon pledget (20) of any of the preceding claims wherein a width of said central portion (23) is less than 60% of a total width of said absorbent material (40).

5. The tampon pledget (20) of any of the preceding claims wherein each of said first and second lateral portions range in width from 5 mm to 80 mm.

6. The tampon pledget (20) of any of the preceding claims wherein a thickness of each of said first and second lateral portions range from 5 mm to 35 mm.

7. The tampon pledget (20) of any of the preceding claims wherein the ratio of said first lateral portion thickness to said central portion thickness is greater than 1.20:1.

8. The tampon pledget (20) of any of the preceding claims wherein the ratio of said first lateral portion thickness to said central portion thickness is from 1.20:1 to 3.50:1.

9. A method of manufacturing a tampon pledget (20) according to claim 1, the method comprising the steps of: a. providing an absorbent material which comprises a first surface, a second surface opposed to said first surface, an insertion end opposed to a withdrawal end, a first longitudinal edge opposed to a second longitudinal edge, a first lateral portion opposed to a second lateral portion, a central portion between said first lateral portion and said second lateral portion; b. providing an overwrap; and c. attaching said overwrap to said central portion of said absorbent material in a, width greater than 3 mm and wherein a thickness of said central portion ranges from 1 mm to 10 mm.

10. The method of Claim 9 wherein said attachment of said overwrap to said central portion of said absorbent material forms at least one region of attachment.

## Patentansprüche

1. Tamponbausch (20), aufweisend:
a. ein Absorbensmaterial (30), das eine erste Oberfläche, eine zweite Oberfläche, die der ersten Oberfläche entgegengesetzt ist, ein Einführungsende (36), das einem Entnahmeende (38) entgegengesetzt ist, einen ersten Längsrand (24), der einem zweiten Längsrand (25) entgegengesetzt ist, einen ersten seitlichen Abschnitt (26), der einem zweiten seitlichen Abschnitt (27) entgegengesetzt ist, und einen mittleren Abschnitt (23) zwischen dem ersten seitlichen Abschnitt (26) und dem zweiten seitlichen Abschnitt (27) umfasst, wobei eine Dicke des mittleren Abschnitts (23) im Bereich von 1 mm bis 10 mm liegt, wobei das Absorbensmaterial (30) eine Länge von 30 bis 100 mm, eine Breite von 40 bis 90 mm und ein Grundgewicht von 250 bis 1100 g/m² aufweist, und
b. eine Umhüllung (40), die die erste Oberfläche des Absorbensmaterials (30) bedeckt,
wobei der Tamponbausch (20) **dadurch gekennzeichnet ist, dass** die Umhüllung (40) am mittleren Abschnitt (23) in einer Breite von mehr als 3 mm befestigt ist.

2. Tamponbausch (20) nach Anspruch 1, wobei die Umhüllung (40) am mittleren Abschnitt (23) anhand eines Verfahrens befestigt wird, das ausgewählt wird aus der Gruppe bestehend aus Prägen, Klebstoff, Anpressen, Wärmebonden, thermisches Bonden, Fusionsbonden, Annähen und Kombinationen davon.

3. Tamponbausch (20) nach einem der vorangehenden Ansprüche, wobei eine Breite des mittleren Abschnitts (23) im Bereich von 3 mm bis 30 mm liegt.

4. Tamponbausch (20) nach einem der vorangehenden Ansprüche, wobei eine Breite des mittleren Abschnitts (23) weniger als 60 % einer Gesamtbreite des Absorbensmaterials (40) ausmacht.

5. Tamponbausch (20) nach einem der vorangehenden Ansprüche, wobei jeder der ersten und zweiten seitlichen Abschnitte eine Breite im Bereich von 5 mm bis 80 mm aufweist.

6. Tamponbausch (20) nach einem der vorangehenden Ansprüche, wobei eine Dicke jedes der ersten und zweiten seitlichen Abschnitte im Bereich von 5 mm bis 35 mm liegt.

7. Tamponbausch (20) nach einem der vorangehenden Ansprüche, wobei das Verhältnis der Dicke des ersten seitlichen Abschnitts zur Dicke des mittleren Abschnitts größer als 1,20 : 1 ist.

8. Tamponbausch (20) nach einem der vorangehenden Ansprüche, wobei das Verhältnis der Dicke des ersten seitlichen Abschnitts zur Dicke des mittleren Abschnitts im Bereich von 1,20 : 1 bis 3,50 : 1 liegt.

9. Verfahren zur Herstellung eines Tamponbausches (20) nach Anspruch 1, wobei das Verfahren folgende Schritte umfasst:
a. Bereitstellen eines Absorptionsmaterials, das eine erste Oberfläche, eine zweite Oberfläche, die der ersten Oberfläche entgegengesetzt ist, ein Einführungsende, das einem Entnahmeende entgegengesetzt ist, einen ersten Längsrand, der einem zweiten Längsrand entgegengesetzt ist, einen ersten seitlichen Abschnitt, der einem zweiten seitlichen Abschnitt entgegengesetzt ist, einen mittleren Abschnitt zwischen dem ersten seitlichen Abschnitt und dem zweiten seitlichen Abschnitt umfasst,
b. Bereitstellen einer Umhüllung und
c. Befestigen der Umhüllung am mittleren Abschnitt des Absorptionsmaterials in einer Breite von mehr als 3 mm, wobei die Dicke des mittleren Abschnitts im Bereich von 1 mm bis 10 mm liegt.

10. Verfahren nach Anspruch 9, wobei die Befestigung der Umhüllung am mittleren Abschnitt des Absorbensmaterials mindestens eine Befestigungsregion bildet.

## Revendications

1. Compresse de tampon (20) comprenant
a. un matériau absorbant (30) qui comprend une première surface, une deuxième surface opposée à ladite première surface, une extrémité d'insertion (36) opposée à une extrémité de retrait (38), un premier bord longitudinal (24) opposé à un deuxième bord longitudinal (25), une première partie latérale (26) opposée à une deuxième partie latérale (27), et une partie centrale (23) entre ladite première partie latérale (26) et ladite deuxième partie latérale (27), et dans laquelle une épaisseur de ladite partie centrale (23) va de 1 mm à 10 mm, ledit matériau absorbant (30) ayant une longueur allant de 30 à 100 mm, une largeur allant de 40 à 90 mm et une masse surfacique allant de 250 à 1100 g/m² ; et
b. un recouvrement (40) qui couvre ladite première surface dudit matériau absorbant (30),
ladite compresse de tampon (20) étant **caractérisée en ce que** ledit recouvrement (40) est fixé à ladite partie centrale (23) dans une largeur supérieure à 3 mm.

2. Compresse de tampon (20) selon la revendication 1, dans laquelle ledit recouvrement (40) est fixé à ladite partie centrale (23) par un procédé choisi dans le groupe constitué de gaufrage, adhésif, liaison par pression, liaison par la chaleur, liaison thermique, liaison par fusion, couture et leurs combinaisons.

3. Compresse de tampon (20) selon l'une quelconque des revendications précédentes, dans laquelle une largeur de ladite partie centrale (23) va de 3 mm à 30 mm.

4. Compresse de tampon (20) selon l'une quelconque des revendications précédentes, dans laquelle une largeur de ladite partie centrale (23) est inférieure à 60 % d'une largeur totale dudit matériau absorbant (40).

5. Compresse de tampon (20) selon l'une quelconque des revendications précédentes, dans laquelle chacune desdites première et deuxième parties latérales va en largeur de 5 mm à 80 mm.

6. Compresse de tampon (20) selon l'une quelconque des revendications précédentes, dans laquelle une épaisseur de chacune desdites première et deuxième parties latérales va de 5 mm à 35 mm.

7. Compresse de tampon (20) selon l'une quelconque des revendications précédentes, dans laquelle le rapport de ladite épaisseur de première partie latérale sur ladite épaisseur de partie centrale est supérieur à 1,20:1.

8. Compresse de tampon (20) selon l'une quelconque des revendications précédentes, dans laquelle le rapport de ladite épaisseur de première partie latérale sur ladite épaisseur de partie centrale va de 1,20:1 à 3,50:1.

9. Procédé de fabrication d'une compresse de tampon (20) selon la revendication 1, le procédé comprenant les étapes consistant à : a. fournir un matériau absorbant qui comprend une première surface, une deuxième surface opposée à ladite première surface, une extrémité d'insertion opposée à une extrémité de retrait, un premier bord longitudinal opposé à un deuxième bord longitudinal, une première partie latérale opposée à une deuxième partie latérale, une partie centrale entre ladite première partie latérale et ladite deuxième partie latérale ; b. fournir un recouvrement ; et c. fixer ledit recouvrement à ladite partie centrale dudit matériau absorbant dans une largeur supérieure à 3 mm et dans lequel une épaisseur de ladite partie centrale va de 1 mm à 10 mm.

10. Procédé selon la revendication 9, dans lequel ledit attachement dudit recouvrement à ladite partie centrale dudit matériau absorbant forme au moins une région d'attachement.
